(19) Europäisches Patentamt
European Patent Office
Office européen des brevets

(11) Veröffentlichungsnummer: **0 302 326 B1**

(12) **EUROPÄISCHE PATENTSCHRIFT**

(45) Veröffentlichungstag der Patentschrift: **15.04.92**

(51) Int. Cl.$^5$: **C07C 25/13**, C07C 17/00

(21) Anmeldenummer: **88111844.2**

(22) Anmeldetag: **22.07.88**

# (54) Verfahren zur Herstellung von mit Fluor und gegebenenfalls zusätzlich Chlor substituierten Benzotrifluoriden und neue Benzotrifluoride.

(30) Priorität: **03.08.87 DE 3725659**

(43) Veröffentlichungstag der Anmeldung:
**08.02.89 Patentblatt 89/06**

(45) Bekanntmachung des Hinweises auf die Patenterteilung:
**15.04.92 Patentblatt 92/16**

(84) Benannte Vertragsstaaten:
**BE CH DE FR GB IT LI NL**

(56) Entgegenhaltungen:
**GB-A- 1 067 412**
**US-A- 4 351 978**
**US-A- 4 590 315**

**CHEMICAL ABSTRACTS, Band 87, Nr. 17, 24. Oktober 1977, Columbus, Ohio, USA; V. E. PLATONOV et al: "Thermolytic reactions of polyfluoroorganic compounds. XIX. Reaction of decafluorobiphenyl and octafluoronaphthalene with potassium fluoride and poly(tetrafluoroethylene)", S. 685, Zusammenfassung Nr. 134761d**

(73) Patentinhaber: **BAYER AG**

**W-5090 Leverkusen 1 Bayerwerk(DE)**

(72) Erfinder: **Marhold, Albrecht, Dr.**
**Carl-Duisberg-Strasse 329**
**W-5090 Leverkusen 1(DE)**
Erfinder: **Braden, Rudolf, Dr.**
**Nothauser Feld 1**
**W-5068 Odenthal(DE)**

**EP 0 302 326 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Rank Xerox (UK) Business Services

## Beschreibung

Die vorliegende Erfindung betrifft ein Verfahren zur Herstellung von mit Fluor und gegebenenfalls zusätzlich Chlor substituierten Benzotrifluoriden durch selektive Hydrierung von Fluor und Chlor enthaltenden Benzotrifluoriden und neue, auf diesem Weg zugängliche Benzotrifluoride.

Fluorierte Benzotrifluoride werden bisher im allgemeinen aus Chlorbenzotrifluoriden hergestellt, indem man bei diesen Chlor beispielsweise mit Kaliumfluorid gegen Fluor austauscht (siehe G. Fuller, J. Chem. Soc. 1965. 6264; J.P. Kolenko et al., Zh. Obsch. Khim. 37, 1686 (1967) und EP-A-34 402). Bei einem derartigen Chlor/Fluor/Austausch lassen sich die aktivierten Chloratome in o- und p-Stellung zur $CF_3$-Gruppe relativ leicht austauschen, während die Cl-Atome in der m-Position nur sehr schwer durch Fluor zu ersetzen sind. Ein bestimmtes Muster der Fluorsubstituenten im gewünschten Produkt verlangt also, daß entsprechende Chlorverbindungen zur Verfügung stehen. Die Chlorierung von Benzotrifluorid verläuft aber in einem durch die dirigierende Wirkung der $CF_3$-Gruppe festgelegten Schema, das es in manchen Fällen unmöglich macht, bestimmte Substitutionsmuster zu erhalten (siehe A.H. Ushakov et al., Zh, Organ. Khim. 12, 2204 (1976) und 20, 2187 (1984)). Dazu zählen beispielsweise das 2,4,6-Trichlorbenzotrifluorid und das 2,3,4,6-Tetrachlorbenzotrifluorid. Es besteht also noch ein Bedarf an einem einfachen Verfahren zur Herstellung von fluorierten Benzotrifluoriden, das diese Schwierigkeiten umgeht.

Es wurde nun ein Verfahren zur Herstellung von mit Fluor und gegebenenfalls zusätzlich Chlor substituierten Benzotrifluoriden gefunden, das dadurch gekennzeichnet ist, daß man Fluor und Chlor enthaltende Benzotrifluoride in Gegenwart eines Katalysators und eines Chlorwasserstoff-Akzeptors hydriert.

In das erfindungsgemäße Verfahren können beispielsweise Fluor und Chlor enthaltende Benzotrifluoride eingesetzt werden, die der Formel (I) entsprechen

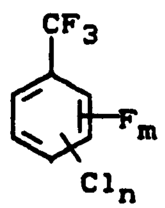

(I),

in der

m für eine ganze Zahl von 1 bis 4 und

n für eine ganze Zahl von 1 bis 5 - m

stehen.

Die Ausgangs-Benzotrifluoride enthalten vorzugsweise 2 bis 4 Fluoratome und 1 bis 2 Chloratome. Die Chloratome befinden sich dabei vorzugsweise in ortho- oder meta-Stellung zur $CF_3$-Gruppe, besonders bevorzugt in meta-Stellung. Besonders bevorzugte Ausgangs-Benzotrifluoride sind 2,3,4,6-Tetrafluor-5-chlor-, 2,4,6-Trifluor-3,5-dichlor-, 2,4-Difluor-3,5-dichlor-, 3,4-Difluor-5-chlor-, 2,3,4-Trifluor-5-chlor-, 4,5-Difluor-2-chlor-, 2,4-Difluor-5-chlor- und 2,4-Difluor-3-chlor-benzotrifluorid.

Geeignete Ausgangs-Benzotrifluoride für das erfindungsgemäße Verfahren können beispielsweise hergestellt werden, indem man Benzotrifluorid zunächst chloriert und dann einen Teil der eingebrachten Chloratome durch Umsetzung mit Kaliumfluorid gegen Fluoratome austauscht (siehe z.B. G. Fuller, J. Chem. Soc. 1965, 6264, J.P. Kolenko et al., Zh. Obsch. Khim. 37, 1686 (1967) und EP-A- 34 402).

Als Katalysatoren kommen im Prinzip bekannte Hydrierkatalysatoren in Frage, beispielsweise solche, welche die Elemente der VIII. Nebengruppe des Periodensystems in metallischer Form oder in Form von Verbindungen enthalten. Insbesondere geeignet sind Nickel, Platin, Palladium und deren Verbindungen, beispielsweise in Form von Raney-Nickel, metallischem Platin, metallischem Palladium und Palladium-tetra-(triphenylphosphin). Die katalytisch aktive Substanz kann auch auf Trägermaterialien aufgebracht sein, beispielsweise auf Aktivkohle, Siliziumdioxid, Aluminiumoxid, Silikaten oder Erdalkalisulfaten.

Bevorzugte Katalysatoren sind Raney-Nickel und metallisches Palladium auf Aktivkohle.

Die Katalysatoren können auch aus mehreren Komponenten zusammengesetzt sein und z.B. auch Promotoren enthalten, bei denen es sich auch um andere Elemente und Verbindungen als diejenigen der Metalle der VIII. Nebengruppe des Periodensystems handeln kann.

Die Menge des Katalysators ist im allgemeinen nicht kritisch. Sie kann beispielsweise 0,01 bis 15 Gew.-%, bezogen auf das eingesetzte Benzotrifluorid betragen. Vorzugsweise beträgt diese Menge 0,1 bis 10 Gew.-%. Unter "Katalysator" wird dabei der aktive Katalysator verstanden, im Falle von Trägerkatalysatoren also nur das aktive Metall.

Die Hydrierung wird im allgemeinen in Gegenwart von Lösungs-oder Verdünnungsmitteln durchgeführt. Bei diesen ist es nicht zwingend erforderlich, daß sie die Einsatzmaterialien vollständig aufzulösen vermögen, da auch ein zweiphasiges Substrat hydriert werden kann. Beispielsweise können das Ausgangs-Benzotrifluorid und/oder der Chlorwasserstoff-Akzeptor während der Hydrierung ganz oder teilweise in suspendierter Form vorliegen. Geeignete Lösungs- und Verdünnungsmittel sind beispielsweise organische Säuren, wie Essigsäure; Alkohole, wie Methanol, Ethanol, Propanol und Isopropanol; Ether, wie Tetrahydrofuran; Nitrile, wie Acetonitril und Wasser.

Als Chlorwasserstoff-Akzeptoren kommen die verschiedensten anorganischen und organischen Basen in Frage, beispielsweise die Hydroxide, Carbonate, Acetate und Ammoniumsalze der Alkali- und Erdalkalimetalle und Amine, insbesondere tertiäre Amine. Bevorzugt sind Natriumacetat und Triethylamin, N,N-Dimethylanilin, Pyrimidin und Picolin.

Der Chlorwasserstoff-Akzeptor kann in verschiedenen Mengen eingesetzt werden. Vorzugsweise werden pro Äquivalent aus dem eingesetzten Benzotrifluorid abzuspaltender Chloratome mindestens 0,8 Äquivalente Chlorwasserstoff-Akzeptor eingesetzt. Wenn aus dem eingesetzten Benzotrifluorid alle vorhandenen Chloratome abgespalten werden sollen ist die Menge des einzusetzenden Chlorwasserstoff-Akzeptors nach oben hin nicht kritisch. Aus praktischen Erwägungen ist es im allgemeinen vorteilhaft, in diesem Fall nicht mehr als 2 Äquivalente Chlorwasserstoff-Akzeptor pro Äquivalent abzuspaltender Chloratome einzusetzen. Wenn aus dem eingesetzten Benzotrifluorid nicht alle vorhandenen Chloratome abgespalten, d.h. noch Chloratome enthaltende Benzotrifluoride hergestellt werden sollen, so darf nicht wesentlich mehr Chlorwasserstoff-Akzeptor eingesetzt werden als stöchiometrisch erforderlich ist. Vorzugsweise setzt man in diesem Fall bis zu 1,2, besonders bevorzugt bis 1,05 und ganz besonders bevorzugt 1 Äquivalent Chlorwasserstoff-Akzeptor pro Äquivalent abzuspaltende Chloratome ein.

Die erfindungsgemäße Hydrierung kann beispielsweise bei Drucken im Bereich von Normaldruck bis 200 bar und Temperaturen im Bereich 20 bis 200 ° C durchgeführt werden. Bevorzugt sind Drucke im Bereich Normaldruck bis 120 bar und Temperaturen im Bereich von 50 bis 140 ° C.

Das Ende der erfindungsgemäßen Reaktion ist im Falle vollständiger Entchlorierung des Einsatzproduktes dann erreicht, wenn kein Wasserstoff mehr aufgenommen wird und im Falle selektiver Entchlorierung, wenn die stöchiometrisch erforderliche Wasserstoffmenge aufgenommen worden ist.

Die Aufarbeitung des Reaktionsgemisches kann auf einfache Weise erfolgen. Beispielsweise kann man zunächst die festen Bestandteile des Reaktionsgemisches (im allgemeinen ist das der Katalysator, gegebenenfalls auch z.B. der beladene Chlorwasserstoff-Akzeptor) durch Filtration abtrennen und aus dem Filtrat durch Destillation das hergestellte, mit Fluor und gegebenenfalls zusätzlich Chlor substituierte Benzotrifluorid gewinnen.

Das erhaltene Benzotrifluorid enthält mindestens 1 Chloratom weniger als das Ausgangs-Benzotrifluorid. Beispielsweise kann es sich bei dem hergestellten Benzotrifluorid um ein Produkt der Formel (II) handeln

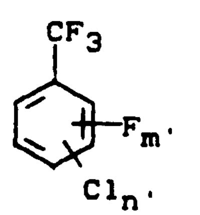

(II),

in der

m′ für eine ganze Zahl von 1 bis 4 und

n′ für Null oder eine ganze Zahl bis zu 4 - m′

stehen.

Das erfindungsgemäße Verfahren hat den Vorteil, daß es in guten Ausbeuten sehr reine Produkte liefert. Die Ausgangsverbindungen sind gut zugänglich, weil der Austausch von Chlor gegen Fluor in höherchlorierten Benzotrifluoriden leichter vonstatten geht als bei niedriger chlorierten Benzotrifluoriden. Der erfindungsgemäße Weg zu Benzotrifluoriden, die mit Fluor und gegebenenfalls Chlor substituiert sind, ist also vorteilhafter als die direkte Einführung von Fluor in unsubstituierte Benzotrifluoride. Außerdem können mit Fluor und gegebenenfalls Chlor substituierte Benzotrifluoride mit Substitutionsmustern erhalten werden, die bisher nicht oder nur schwer zugänglich waren.

Überraschend beim erfindungsgemäßen Verfahren ist, daß die am aromatischen Kern vorhandenen Fluor-Substituenten nicht abgespalten werden und auch praktisch keine Hydrierung des aromatischen Kerns

auftritt. Außerdem ist überraschend, daß beim Vorhandensein mehrerer Chlor-Substituenten im Ausgangsprodukt diese einzeln in guten Selektivitäten abgespalten werden können.

Die vorliegende Erfindung betrifft weiterhin mit Fluor substituierte Benzotrifluoride der Formel (III)

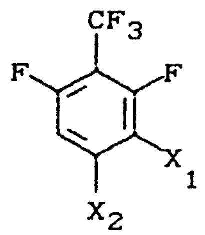

(III),

in der

$X_1$ für Fluor oder Chlor und

$X_2$ im Falle von $X_1$ = Fluor für Wasserstoff und im Falle von $X_1$ = Chlor für Fluor

stehen.

Insbesondere handelt es sich bei den Benzotrifluoriden der Formel (III) um 2,4,6-Trifluor-3-chlorbenzotrifluorid und 2,3,6-Trifluor-benzotrifluorid.

Die Benzotrifluoride der Formel (III) können wie weiter oben beschrieben hergestellt werden.

Mit Fluor und gegebenenfalls Chlor substituierte Benzotrifluoride sind wichtige Zwischenprodukte zur Herstellung von Wirkstoffen. Beispielsweise kann man gemäß der vorliegenden Erfindung erhältliche, mit Fluor und gegebenenfalls Chlor substituierte Benzotrifluoride mit Hydrazinhydrat reagieren lassen und die dabei erhältlichen Arylhydrazine mit 1,3-Diketonen der Formel (IV)

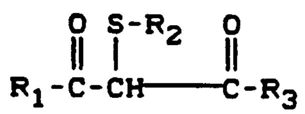

(IV),

umsetzen, in der

$R_1$ und $R_3$ für Alkyl und

$R_2$ für Alkyl oder Halogenalkyl stehen,

und so 3,5-Dialkyl-1-aryl-pyrazole der Formel (V)

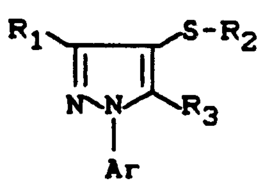

(V),

erhalten, in der

$R_1$ bis $R_3$ die Formel (IV) angegebene Bedeutung haben und

Ar für einen fluorierten und gegebenenfalls chlorierten Benzotrifluoridrest

steht.

Aus Verbindungen der Formel (V) lassen sich durch Oxidation in üblicher Weise Verbindungen der Formel (VI)

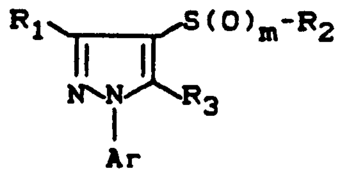

(VI),

herstellen, in der

| | |
|---|---|
| $R_1$ bis $R_3$ und Ar | die bei Formel (V) angegebene Bedeutung haben und |
| m | für 1 oder 2 steht. |

Die Verbindungen der Formeln (V) und (VI) zeichnen sich durch eine hohe insektizide Wirksamkeit aus, beispielsweise gegen pflanzenschädigende Insekten wie die Larven des Meerrettichblattkäfers. Sie besitzen auch eine hohe Wirkung gegen Hygiene- und Vorratsschädlinge wie die Stubenfliege (Musca domestica) und gegen parasitisch lebende Warmblüterschädlinge wie die Weideviehfliege (Musca autumnalis).

## Beispiele

### Beispiel 1

In einer Hydrierapparatur wurden 1 Mol Ausgangsmaterial in 1 l Essigsäure zusammen mit 1,05 Mol Natriumacetat pro Mol abzuspaltendem Chlor vorgelegt. Dann wurden 10 g Katalysator (5 Gew.-% metallisches Palladium auf Aktivkohle) zugegeben und anschließend bei einem Wasserstoffdruck von 30 bis 50 bar bei 120 °C hydriert bis der Druck konstant blieb. Danach wurde aus dem Reaktionsgemisch der Katalysator durch Filtration abgetrennt und das Filtrat über eine Kolonne destilliert. Das Destillat wurde mit Wasser gewaschen, um kleine Mengen Essigsäure zu entfernen. Das Reaktionsprodukt hatte jeweils eine Reinheit von über 98 %. Weitere Details sind aus der Tabelle 1 ersichtlich.

Tabelle 1

| Beispiel Nr. | Ausgangsmaterial (...-benzotrifluorid) | Produkt (...benzotrifluorid) | Siedepunkt bei 1013 mbar | Brechungsindex $n_D^{20}$ | Ausbeute % d.Th. |
|---|---|---|---|---|---|
| 1a | 5-Chlor-tetrafluor- | 2,3,4,6-Tetrafluor- | 105-106 °C | 1.3770 | 92 |
| 1b | 3,5-Dichlor-trifluor- | 2,4,6-Trifluor- | 104 °C | 1.3840 | 83 |
| 1c | 3,5-Dichlor-trifluor- | 3-Chlor-2,4,6-trifluor- | 145 °C | 1.4170 | 76 |
| 1d | 3,5-Dichlor-2,4-difluor- | 2,4-Difluor- | 105-106 °C | 1.3968 | 84 |
| 1e | 5-Chlor-3,4-difluor- | 3,4-Difluor- | 103-104 °C | 1.3958 | 85 |
| 1f | 5-Chlor-2,3,4-trifluor- | 2,3,4-Trifluor- | 105-106 °C | 1.3842 | 89 |
| 1g | 3-Chlor-2,5,6-trifluor- | 2,3,6-Trifluor- | 105-107 °C | 1.3850 | 82 |

### Beispiel 2

In einer Hydrierapparatur wurden 216 g (1 Mol) 2-Chlor-4,5-difluor-benzotrifluorid in 1 l Methanol vorgelegt, 110 g Triethylamin und 15 g Raney-Nickel zugegeben. Danach wurde die Hydrierapparatur mit Wasserstoff gespült, dann auf 80 °C erwärmt und 100 bar Wasserstoff aufgedrückt. Nach Abnahme des Wasserstoffdruckes wurde erneut auf 100 bar ergänzt und bis zur Druck-Konstanz reagieren gelassen. Nach dem Abkühlen wurde der Druck entspannt und das Reaktionsgemisch filtriert. Das Filtrat wurde mit 3 l Wasser verdünnt und die organische Phase abgetrennt. Diese wurde getrocknet und über eine Kolonne destilliert. Es wurden 147 g 3,4-Difluor-benzotrifluorid mit einem Siedepunkt bie 1013 mbar von 108 bis 109 °C und einem Brechungsindex $n_D^{20}$ von 1,3973 erhalten.

### Beispiel 3

Es wurde verfahren wie in Beispiel 2, jedoch wurde 1 Mol 5-Chlor-2,4-difluor-benzotrifluorid eingesetzt und 151 g 2,4-Difluor-benzotrifluorid mit einem Siedepunkt bei 1013 mbar von 105 bis 106 °C und einem Brechungsindex $n_D^{20}$ von 1,3970 erhalten.

Beispiel 4

In einer Hydrierapparatur mit Gaseinleitungsfritte und Rührer wurden 100 g 3-Chlor-2,4-difluor-benzotrifluorid in 500 ml Acetonitril und 60 g gepulvertem Natriumcarbonat vorgelegt. Dann wurden 10 g Katalysator (5 Gew.-% metallisches Palladium auf Aktivkohle) zugegeben und bei 80 ° C 10 Stunden lang Wasserstoff eingeleitet. Danach wurde der Ansatz abgekühlt, filtriert und das Filtrat über eine mit Edelstahl-Siebringen gefüllte Kolonne destilliert. Es wurden 56 g 2,4-Difluor-benzotrifluorid mit einem Siedepunkt von 105 bis 106 ° C bei 1030 mbar erhalten. Der Brechungsindex $n_D^{20}$ betrug 1.3968.

Beispiel 5

In einer Hydrierapparatur wurden 300 g 3,5-Dichlor-2,6-difluorbenzotrifluorid in 1200 ml Essigsäure und 210 g Natriumacetat vorgelegt, 10 g 5 %-iges Palladium auf A-Kohle zugegeben und nach Spülen mit Wasserstoff mit einem Wasserstoff-Druck von 30 bis 50 bar bei 80 - 120 ° C hydriert. Nach Ende der Wasserstoffaufnahme (Druckkonstanz) wurde abgekühlt, der Katalysator abgesaugt und die Lösung mit 2 l Wasser verrührt. Die sich abscheidende organische Phase wurde abgetrennt und destilliert. Siedepunkt: 106 - 107 ° C; $n_D^{20}$ : 1.3972; Ausbeute: 193 g an 2,6-Difluorbenzotrifluorid.

**Patentansprüche**

1. Verfahren zur Herstellung von mit Fluor und gegebenenfalls zusätzlich Chlor substituierten Benzotrifluoriden, dadurch gekennzeichnet, daß man mit Fluor und Chlor substituierte Benzotrifluoride in Gegenwart eines Katalysators und eines Chlorwasserstoff-Akzeptors hydriert.

2. Verfahren nach Anspruch 1, dadurch gekennzeichnet, daß die eingesetzten Benzotrifluoride der Formel (I) entsprechen

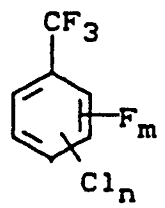

(I),

in der

m für eine ganze Zahl von 1 bis 4 und

n für eine ganze Zahl von (1 bis 5) - m

stehen.

3. Verfahren nach Ansprüchen 1 und 2, dadurch gekennzeichnet, daß der Katalysator Elemente der VIII. Nebengruppe des Periodensystems in metallischer Form oder in Form von Verbindungen enthält.

4. Verfahren nach Ansprüchen 1 bis 3, dadurch gekennzeichnet, daß man in Gegenwart von Lösungs- oder Verdünnungsmitteln arbeitet.

5. Verfahren nach Ansprüchen 1 bis 4, dadurch gekennzeichnet, daß man in Gegenwart von Hydroxiden, Carbonaten, Acetaten und Ammoniumsalzen der Alkali- und Erdalkalimetalle und/oder in Gegenwart von Aminen als Chlorwasserstoff-Akzeptoren hydriert.

6. Verfahren nach Ansprüchen 1 bis 5, dadurch gekennzeichnet, daß man pro Äquivalent abzuspaltender Chloratome mindestens 0,8 Äquivalente Chlorwasserstoff-Akzeptor einsetzt.

7. Verfahren nach Ansprüchen 1 bis 6, dadurch gekennzeichnet, daß man bei Drucken im Bereich von Normaldruck bis 200 bar und bei Temperaturen im Bereich von 20 bis 200 ° C hydriert.

8. Fluorierte Benzotrifluoride der Formel (III)

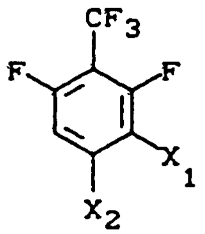

(III),

in der

$X_1$ für Fluor oder Chlor und

$X_2$ im Falle von $X_1$ = Fluor für Wasserstoff und im Falle von $X_1$ = Chlor für Fluor

stehen.

**9.** 2,4,6-Trifluor-3-chlor-benzotrifluorid und 2,3,6-Trifluor-benzotrifluorid.

## Claims

**1.** Process for the preparation of benzotrifluorides which are substituted by fluorine and optionally in addition by chlorine, characterised in that benzotrifluorides which are substituted by fluorine and chlorine are hydrogenated in the presence of a catalyst and in the presence of a hydrogen chloride acceptor.

**2.** Process according to Claim 1, characterised in that the benzotrifluorides employed correspond to the formula (I)

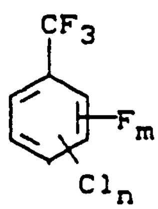

(I),

in which

m represents an integer from 1 to 4 and

n represents an integer from (1 to 5) - m.

**3.** Process according to Claims 1 and 2, characterised in that the catalyst contains elements of the subgroup VIII of the periodic table in the form of metals or in the form of compounds.

**4.** Process according to Claims 1 to 3, characterised in that the process is carried out in the presence of solvents or diluents.

**5.** Process according to Claims 1 to 4, characterised in that the hydrogenation is carried out in the presence of hydroxides, carbonates, acetates and ammonium salts of alkali metals and alkaline earth metals, and/or in the presence of amines as hydrogen chloride acceptors.

**6.** Process according to Claims 1 to 5, characterised in that at least 0.8 equivalent of hydrogen chloride acceptor is employed per equivalent of chlorine atoms to be eliminated.

**7.** Process according to Claims 1 to 6, characterised in that the hydrogenation is carried out at pressures in the range from atmospheric pressure to 200 bar and at temperatures in the range from 20 to 200 °C.

**8.** Fluorinated benzotrifluorides of the formula (III)

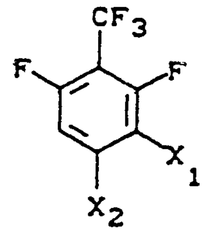 (III),

in which

$X_1$ represents fluorine or chlorine and

$X_2$ in the case of $X_1$ = fluorine, represents hydrogen, and, in the case of $X_1$ = chlorine, represents fluorine.

**9.** 2,4,6-Trifluoro-3-chlorobenzotrifluoride and 2,3,6-trifluorobenzotrifluoride.

**Revendications**

**1.** Procédé de préparation de benzotrifluorures substitués par le fluor et éventuellement par le chlore, caractérisé en ce que l'on hydrogène des benzotrifluorures substitués par le fluor et le chlore en présence d'un catalyseur et d'un accepteur du chlorure d'hydrogène.

**2.** Procédé selon la revendication 1, caractérisé en ce que les benzotrifluororures mis en oeuvre répondent à la formule I

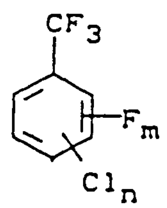 (I),

dans laquelle

m est un nombre entier allant de 1 à 4 et

n est un nombre entier allant de (1 à 5) - m.

**3.** Procédé selon les revendications 1 et 2, caractérisé en ce que le catalyseur contient des éléments du sous-groupe VIII de la Classification Périodique à l'état métallique ou à l'état de composés.

**4.** Procédé selon les revendications 1 à 3, caractérisé en ce que l'on opère en présence de solvants ou diluants.

**5.** Procédé selon les revendications 1 à 4, caractérisé en ce que l'on hydrogène en présence d'hydroxydes, carbonates, acétates et sels d'ammonium des métaux alcalins et alcalino-terreux et/ou en présence d'amines servant d'accepteurs du chlorure d'hydrogène.

**6.** Procédé selon les revendications 1 à 5, caractérisé en ce que l'on utilise au moins 0,8 équivalent de l'accepteur du chlorure d'hydrogène par équivalent des atomes de chlore à éliminer.

**7.** Procédé selon les revendications 1 à 6, caractérisé en ce que l'on hydrogène à des pressions dans l'intervalle allant de la pression normale jusqu'à 200 bar et à des températures situées dans l'intervalle de 20 à 200 °C.

**8.** Benzotrifluorures fluorés de formule III

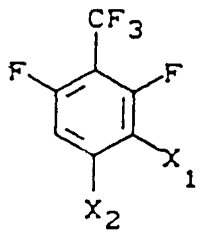

(III),

dans laquelle
$X_1$ représente le fluor ou le chlore et
$X_2$, dans le cas où $X_1$ représente le fluor, représente l'hydrogène, et, dans le cas où $X_1$ représente le chlore, il représente le fluor.

**9.** Le 2,4,6-trifluoro-3-chlorobenzotrifluorure et le 2,3,6-trifluorobenzotrifluorure.